# EUROPEAN PATENT SPECIFICATION

(11) **EP 2 790 695 B1**
(45) Date of publication and mention of the grant of the patent: **16.03.2016**
(21) Application number: 12798303.9
(22) Date of filing: 10.12.2012
(51) Int. Cl.: A61K 31/4015, A61K 9/16, A61P 25/08

(54) **PHARMACEUTICAL COMPOSITION COMPRISING (S)-2-(2-OXOPYRROLIDIN-1-YL)BUTANAMID**
PHARMACEUTISCHE ZUSAMMENSETZUNG ENTHALTEND (S)-2-(2-OXOPYRROLIDIN-1-YL)BUTANAMID
COMPOSITION PHARMACEUTIQUE COMPRENANT DU (S)-2-(2-OXOPYRROLIDIN-1-YL)BUTANAMID

(30) Priority: 16.12.2011 EP 11009922
(43) Date of publication of application: 22.10.2014
(73) Proprietor: Uni-Pharma Kleon Tsetis Pharmaceutical Laboratories S.A., 145 64 Kifissia (GR)
(72) Inventor: TSETI, Ioulia, GR-14561 Kifissia (GR)
(74) Representative: Wibbelmann, Jobst
(86) International application number: PCT/EP2012/074931
(87) International publication number: WO 2013/087563

(56) References cited:
- WO-A1-2010/006929
- WO-A2-2009/087675
- WO-A2-2011/136751
- US-A1- 2010 172 979

## Description

The present invention relates to a solid pharmaceutical composition comprising (S)-2-(2-oxopyrrolidin-1-yl)butanamid, in particular to granules comprising (S)-2-(2-oxopyrrolidin-1-yl)butanamid, and to the preparation thereof.

### Background prior art

(S)-2-(2-oxopyrrolidin-1-yl)butanamid (CAS Registry Number 102767-28-2), also known as (-)-(S)-a-ethyl-2-oxo-1-pyrrolidine acetamide or Levetiracetam, is an antiepileptic drug. Its molecular formula is C₈H₁₄N₂O₂ and the molecular weight is 170,21 g/mol.

KEPPRA^{®}, the current brand Levetiracetam, is available in tablets of 500 mg and 1000 mg concentration and in liquid formulation as oral solution, which contains 100 mg of (S)-2-(2-oxopyrrolidin-1-yl)butanamid per mL, which use as excipients ammonium glycyrizzinate, sugar and parabens. Due to the sugar, the composition is less suitable for diabetic patients. Maltitol is used as sweetener for masking the bitter and unpleasant taste of (S)-2-(2-oxopyrrolidin-1-yl)butanamid, wherein maltitol needs the presence of parabens as antimicrobiological preservative. However, parabens are known to cause allergic reactions (see e.g. Handbook of Pharmaceutical Excipients, 3rd edition, page 342 & 452).

US2011/0021786 A1 discloses aqueous solutions of 2-oxo-1-pyrrolidine derivatives. These aqueous solutions suffer from the draw back that they are partially unstable due to basic hydrolysis, acid hydrolysis and oxidation. The amount of degradation can, however, be reduced by keeping the pH value of the solution between 4.5 and 6.5. Solid dosage forms are not disclosed.

WO 2010/006929 A1 discloses various compositions comprising *inter alia* levetiracetam. This composition is provided as a dry syrup.

WO 2011/136751 A2 relates to a water soluble pharmaceutical composition which comprises levetiracetam, in particular as an effervescent tablet composition. An effervescent tablet composition requires the presence of pharmaceutically acceptable acids and bases such that the tablet when it comes into contact with water effervesces. Accordingly, this documents requires that the composition comprises at least one acidic agent in the range of 25% to 60% by weight and at least one basic agent in the range of 10% to 25% by weight. The presence of both acidic and a basic agents impact the stability of the pharmaceutical composition. In any event, the present invention avoids the use of basic agents in an amount necessary to provide effervescent properties (see the limits for acidic and basic agents above).

US 2010/0172979 A1 concerns controlled-release formulations which may comprise levetiracetam. The present invention neither relates to controlled- (or extended-) release levetiracetam tablets. In such a case, the tablet would comprise additional components which are not water soluble, such as carnauba wax and stearic acid. The present invention avoids the use of non-water-soluble components. In any event, the granules of the present invention can be used as such and need not be compacted into tablets such as the tablets disclosed in the prior art.

It is well known that a water-soluble formulation in the form of tablets yields a solution that can be readily taken and which is more favorable to users by facilitating convenient transportation, as opposed to an equivalent formulation of the active ingredient as an oral solution contained in glass bottles or in vials. Thus, there is a constant search for new solid pharmaceutical compositions comprising (S)-2-(2-oxopyrrolidin-1-yl)butanamid, which overcome the draw backs of the prior art.

Therefore, it was an object of the present invention to provide a solid pharmaceutical composition comprising (S)-2-(2-oxopyrrolidin-1-yl)butanamid, which is free of parabens.

It was another object of the present invention to provide a solid pharmaceutical composition comprising (S)-2-(2-oxopyrrolidin-1-yl)butanamid, which is free of sugars.

It was a further object of the present invention to provide a solid pharmaceutical composition comprising (S)-2-(2-oxopyrrolidin-1-yl)butanamid, which is water-soluble and results in an oral solution when dissolved in water.

It was a further object of the present invention to provide a solid pharmaceutical composition comprising (S)-2-(2-oxopyrrolidin-1-yl)butanamid, which does not need to be pressed into tablets.

It was a further object of the present invention to provide a solid pharmaceutical composition comprising (S)-2-(2-oxopyrrolidin-1-yl)butanamid, which shows improved stability compared to known oral solutions comprising (S)-2-(2-oxopyrrolidin-1-yl)butanamid.

It was another object of the present invention to provide a process for the preparation of a solid pharmaceutical composition comprising (S)-2-(2-oxopyrrolidin-1-yl)butanamid.

### Summary of the Invention

According to one aspect the present invention relates to a solid pharmaceutical composition comprising non-effervescent and water-soluble granules which comprise (S)-2-(2-oxopyrrolidin-1-yl)butanamid and at least one pharmaceutically acceptable acid.

According to a further aspect of the present invention there are provided granules comprising (S)-2-(2-oxopyrrolidin-1-yl)butanamid for use in a medical treatment, in particular for use in a method for treating epilepsy.

According to another aspect of the present invention there are provided granules comprising (S)-2-(2-oxopyrrolidin-1-yl)butanamid for use as an antiepileptic drug.

In still another aspect, there is provided a method of producing granules comprising (S)-2-(2-oxopyrrolidin-1-yl)butanamid, which comprises combining the components of the granules.
The present invention represents an improvement over known solid pharmaceutical compositions comprising (S)-2-(2-oxopyrrolidin-1-yl)butanamid. In particular, there is provided a solid pharmaceutical composition comprising (S)-2-(2-oxopyrrolidin-1-yl)butanamid in the form of water-soluble granules, which are free of sugars and parabens and may allow the formation of a ready to use oral solution comprising an effective amount of (S)-2-(2-oxopyrrolidin-1-yl)butanamid after dissolution of the granules in an appropriate amount of water.

Furthermore, the granules include the ability to overcome the stability issues. In particular, they may be stored under inert gas right after the production thereby avoiding degradation. In addition, as the (S)-2-(2-oxopyrrolidin-1-yl)butanamid may be administered immediately after the dissolution of the granules, there is no need the stabilize the solution by using parabens.

A further advantage may be that granules may be prepared by simply combining the components, which results in a more economical process. Pressing into tablets is not necessary. Additionally, the taste of the solution after dissolving the granules in water is pleasant, without the need of sugars.

### Definitions

The terms "granule" and "granules" as used herein shall be understood as known in the art. In particular, granules are particles gathered into a larger, permanent aggregate in which the original particles can still be identified. The term granulation refers to the act or process in which primary powder particles are made to adhere to form larger, multiparticle entities called granules. The granules as defined herein represent a pharmaceutical composition, particularly a solid pharmaceutical composition.

The terms "tablet" and "tablets" as used herein shall be understood as known in the art. In particular, tablets are a solid dosage form. They comprise a mixture of components, usually in powder form, pressed or compacted from a powder or granules into a solid dose.

The terms "lubricant" and "lubricants" as used herein shall be understood as known in the art. In particular, lubricants are added to decrease friction and to prevent sticking between the components and the processing means during production of a pharmaceutical composition.

The term "sugar" as used herein shall be understood as known in the art. In particular, the pharmaceutical composition or the granules as defined herein are free of monosaccharides and disaccharides referred to as sugars in the art such as glucose, fructose, sucrose, maltose and lactose. Sugar alcohols do not belong to the group of sugars.

The terms "filler" and "fillers" as used herein shall be understood as known in the art. In particular, they are defined as increasing the weight and/or size of a solid pharmaceutical composition.

The terms "sweetener" and "sweeteners" as used herein shall be understood as known in the art. In particular, the sweeteners used herein are sugar substitutes. They may be artificial or natural sweeteners. The sweeteners as defined herein do not include maltitol.

The term "flavoring agent" as used herein shall be understood as known in the art. In particular, a flavoring agent can mask bitter tasting components and/or is added to improve the taste of a pharmaceutical composition.

All amounts and weight % as provided herein refer to the total weight of the respective pharmaceutical composition or granules.

The term "comprising" as used herein shall in every embodiment, aspect, example, item or claim be interpreted as encompassing all the specifically mentioned features, components or steps as well optional, additional, unspecified ones. In addition, the term "comprising" as used herein shall also be interpreted to mean "consisting", therewith defining for every embodiment, aspect, example, item or claim that no further features, components or steps are present.

The terms "ambient temperature" and "room temperature" used herein will be understood be the person skilled in the art as referring to a temperature between about 20 °C and about 25 °C, particularly between 20 °C and 25 °C.

### Detailed Description of the Invention

According to one aspect the present invention relates to granules comprising (S)-2-(2-oxopyrrolidin-1-yl)butanamid and at least one pharmaceutically acceptable acid.
In particular, the present invention relates to a pharmaceutical composition in form of granules comprising (S)-2-(2-oxopyrrolidin-1-yl)butanamid and at least one pharmaceutically acceptable acid.

The acid may be a carboxylic acid, preferably having from 2 to 12, more preferably 5 to 12, and even more preferably 6 to 7 carbon atoms. The carboxylic acid may be a mono-, di-or tri carboxylic acid. Typically, the acid is selected from citric acid, benzoic acid, sorbic acid, succinic acid, tartaric acid, lactic acid, acetic acid, or any combination thereof. Citric acid is preferred. The acid may also be a mineral acid, and is preferably phosphoric acid.

The acid typically plays the role of a pH value regulator after the granules are dissolved in water. The presence of a buffer system could be used supplementary for taste improvement. Typically, the acid or buffer system is present in anhydrous form and is preferably anhydrous citric acid or monosodium citrate. Alternatively, tartaric acid can be used.

The acid may be present in the granules in an amount of 5 to 20 wt.-%, particularly 10 to 15 wt.-%, and more particularly 13 to 14 wt.-%.

The granules as defined herein typically comprise (S)-2-(2-oxopyrrolidin-1-yl)butanamid in an amount of 20 to 50 wt.-%, particularly 30 to 40 wt.-%, and more particularly 33 to 35 wt.-%.

The granules may further comprise at least one lubricant. The lubricant or the lubricants is/are typically selected from polyethylene glycol (PEG) types and sodium benzoate micronized, that are identical for water soluble formulations, or any combination thereof. Polyethylene glycol 4000 and polyethylene glycol 6000 are preferred. A particularly preferred lubricant is polyethylene glycol 6000. Usually, the lubricant is present in the granules in an amount of 1 to 10 wt.-%, particularly 2 to 5 wt.-%, and more particularly 3 to 4 wt.-%.

The granules may further comprise at least one filler. The filler or the fillers is/are typically selected from sorbitol, mannitol, or any combination thereof. Sorbitol or mannitol is preferred. Usually, the filler is present in the granules in an amount of 35 to 60 wt.-%, particularly 40 to 55 wt.-%, and more particularly 48 to 50 wt.-%.

The granules may further comprise at least one sweetener. The sweetener or the sweeteners is/are typically selected from sodium saccharinate, calcium saccharinate, acesulfame potassium, sodium cyclamate, aspartame, or any combination thereof. Usually, the sweetener is present in the granules in an amount of 0.1 to 1 wt.-%, particularly 0.6 to 0.9 wt.-%, and more particularly 0.7 to 0.8 wt.-%.

The granules may further comprise at least one flavoring agent. The flavoring agent or the flavoring agents is/are typically selected from lemon juice flavor, cherry flavor, orange flavor, mandarin flavor, raspberry flavor, strawberry flavor, or any combination thereof. Usually, the flavoring agent is present in the granules in an amount of 0.5 to 3 wt.-%, particularly 1 to 2 wt.-%, and more particularly 1.7 to 1.9 wt.-%.

The granules typically do not comprise maltitol. Preferably, the granules do not comprise sugars such as sucrose, fructose or lactose. Furthermore, the granules typically do not comprise maltodextrine.

The granules as defined herein are typically water soluble. Preferably, they are used for
oral administration, preferably as oral solution in water.

The granules as defined herein typically have a particle size range from 40 µm to 800 µm (determined by a dry sieving method, EUR PH 2.9.38). The particle size distribution within this range, expressed as percent by weight passing through a specific sieve, is as follows:

| **Sieve opening (µm)** | **Percent by weight passing (%)** |
|---|---|
| 750 | 98 |
| 250 | 45 |
| 40 | 2 |

According to one embodiment, the granules comprise or consist of (S)-2-(2-oxopyrrolidin-1-yl)butanamid, a pharmaceutically acceptable acid, a filler, a lubricant, a sweetener, and a flavoring agent.

According to a preferred embodiment, the granules comprise or consist of (S)-2-(2-oxopyrrolidin-1-yl)butanamid, anhydrous citric acid, sorbitol, polyethylene glycol 6000, sodium saccharinate, and lemon juice flavor.

According to a particularly preferred embodiment, the granules consist of 33.3 wt.-% (S)-2-(2-oxopyrrolidin-1-yl)butanamid, 13.3 wt.-% anhydrous citric acid, 47.7 wt.-% sorbitol, 3.3 wt.-% polyethylene glycol 6000, 0.7 wt.-% sodium saccharinate, and 1.7 wt.-% lemon juice flavor.

According to another aspect, the granules as defined herein are used in a medical treatment, in particular in a method for treating epilepsy. In particular, the granules are used as an antiepileptic drug.

Typically, the granules are administered as oral solution immediately after the dissolution of the granules. A suitable amount of water is typically around 100 ml. The resulting pH value is typically around 2.5 to 5.0, preferably 2.5 to 3.5.

### Method for the preparation of the granules as defined herein

According to a further aspect, there is provided a method of producing the granules as defined herein, comprising combining the components of the granules in a combining step. Thereby, the granules are formed.

After the combining step, the granules may be filled in at least one container under an inert gas atmosphere. The container/s may be selected from a sachet, a case, a tube, a sleeve, a bag, a bottle, an ampoule, a blister, a cap, or a foil. Sachets are preferred. The inert gas is usually selected from nitrogen gas, helium gas, argon gas, or any combination thereof and is preferably nitrogen gas.

According to one embodiment, the components are combined in one combining step. According to another embodiment, they are combined in at least two consecutive combining steps, particularly in three consecutive combining steps.

According to a further embodiment, the method of producing the granules as defined herein comprises:
a first combining step, in which (S)-2-(2-oxopyrrolidin-1-yl)butanamid, the at least one pharmaceutically acceptable acid, preferably one/ones as defined herein, and optionally a filler, preferably one/ones as defined herein, are combined to provide a first mixture;
an optional second combining step, in which the first mixture is combined with at least one flavoring agent, preferably one/ones as defined herein, and/or with at least one sweetener, preferably one/ones as defined herein, to provide a second mixture; and
an optional third combining step, in which the second mixture is combined with at least one lubricant, preferably one/ones as defined herein.

The first combining step typically takes 10 to 30 min, particularly 15 to 25 min, and more particularly 20 to 22 min. The at least one pharmaceutically acceptable acid and/or the optionally added filler may be sieved before they are combined in the first combining step, particularly through a 20 mesh sieve.

The combining in the second combining step typically takes 5 to 25 min, particularly 10 to 20 min, and more particularly 15 to 17 min. The flavoring agent and/or the sweetener may be sieved before they are combined in the second combining step, particularly through a 40 mesh sieve.

The combining in the third combining step typically takes 1 to 10 min, particularly 3 to 8 min, and more particularly 5 to 7 min.

Usually, the combining is carried out by mixing, wherein every mixer is suitable which allows formation of granules. Preferably, the components are mixed at 40 rpm during each combining step. Examples for suitable mixers are conical mixers, V mixers or cylindrical mixers.

The granules as defined herein are preferably produced by the method as described herein. However, a person skilled in the art will understand that the granules can also be prepared by other methods, particularly other granulation methods such as wet granulation.

The invention will be more fully understood by references to the following examples.

### Examples

### Example 1: Formulation 1

| Ingredients: | Content: |
|---|---|
| (S)-2-(2-oxopyrrolidin-1-yl)butanamid | 500 mg |
| Citric acid anhydrous | 200 mg |
| Sorbitol | 715 mg |
| Polyethylene glycol 6000 | 50 mg |
| Sodium saccharinate | 10 mg |
| Lemon juice flavor | 25 mg |

### Example 2: Formulation 2

| Ingredients: | Content: |
|---|---|
| (S)-2-(2-oxopyrrolidin-1-yl)butanamid | 1000 mg |
| Citric acid anhydrous | 400 mg |
| Sorbitol | 1430 mg |
| Polyethylene glycol 6000 | 100 mg |
| Sodium saccharinate | 20 mg |
| Lemon juice flavor | 50 mg |

### Example 3: Comparative example, KEPPRA oral solution

| Ingredients: | Content: |
|---|---|
| Levetiracetam | 100 mg/ml |
| Ammonium Glycyrrhizinate | unknown |
| Citric Acid monohydrate | unknown |
| Sodium Citrate dehydrate | unknown |
| Glycerine | unknown |
| Maltitol solution | unknown |
| Methylparaben | unknown |
| Propylparaben | unknown |
| Potassium acesulfame | unknown |
| Natural and artificial flavor | unknown |
| Purified water | unknown |

### Example 4: Method for producing granules

### Step 1

(S)-2-(2-oxopyrrolidin-1-yl)butanamid, anhydrous citric acid and sorbitol are mixed at the first step. The three components are sieved through a 20mesh sieve and they are added into a mixer. The mixture of the three components is mixed for 20min.

### Step 2

The second step includes the addition of the flavor and the sweetener into the mixture of step 1. Lemon juice flavor and sodium saccharinat, are sieved through a 40mesh sieve. The sieved components are added into the mixture of step 1. The whole mixture is mixed for 15min.

### Step 3

The third step includes the addition of the lubricant into the mixture of step 2. Polyethylene glycol 6000 is added into the mixture of step 2. The whole mixture is mixed for 5min.

### Step 4

After the end of step 3 the mixing process is completed and the produced mixture is ready for the packaging procedure. The final mixture is filled in sachets and the sachets are sealed. During the filling procedure, nitrogen gas (N₂) is blown into the sachets, removing the existing oxygen and creating a nitrogen atmosphere. So, the filled sachets are sealed in a nitrogen atmosphere. The sealed sachets are packed in boxes.

### Example 5: Stability measurements

The following table I shows a comparison between the granules of example 1 and the oral solution of comparative example 3.

**Table I**

| Sum of all degradation products detected (% relative areas) after 4 and 8 weeks of stability at 25 °C, 40 °C, 60 °C, 80 °C. (product in aluminium foil) | | | |
|---|---|---|---|
| **4 weeks** | | | |
| Example 1 | | | |
| 25 °C | 40 °C | 60 °C | 80 °C |
| 0.0 | 0.0 | 0.02 | 0.05 |

| Comparative example 3 | | | |
|---|---|---|---|
| 25 °C | 40 °C | 60 °C | 80 °C |
| 0.0 | 0.12 | 0.15 | 1.2 |

| **8 weeks** | | | |
|---|---|---|---|
| Example 1 | | | |
| 25 °C | 40 °C | 60 °C | 80 °C |
| 0.0 | 0.0 | 0.04 | 0.10 |

| Comparative example 3 | | | |
|---|---|---|---|
| 25 °C | 40 °C | 60 °C | 80 °C |
| 0.0 | 0.15 | 0.45 | 3.5 |

These results demonstrate clearly that the degradation rate is lower for the granules of example 1 than for the oral solution of comparative example 3.

### Experimental setup

The determination of the degradation products is performed according to the European Pharmacopoeia 7.1 (published in 2010, 7th edition (supplement 7.1), ISBN number PUB100003**.** The degradation products have been detected by means of LC chromatography. The analysis was carried out by an isocratic mode, at room temperature.

In order to detect one of the typical degradation products, [(2R)-2-(2-oxopyrrolidin-1-yl)butanamide ((R) -etiracetam) called Imp D, the following conditions were used (Chiral HPLC Method) (Table II).

**Table II**

| | |
|---|---|
| Mobile phase | 2-propanol,hexane (18:82 v/v) |
| Flow rate | 0,8 ml/min |
| Detection | Spectrophotometer at 205 nm |
| Column | Silica gel OD for chiral separations I=0,25 m, ∅ =4,6 mm |
| Injection | 20 µl |
| Run Time | 1,4 times the retention time of (S)-2-(2-oxopyrrolidin-1-yl)butanamid |

### Preparation of standard solutions and samples

A test solution is prepared by dissolving 2 ml of KEPPRA solution or M mg of product which is equivalent to 0,200 g of (S)-2-(2-oxopyrrolidin-1-yl)butanamid in 2-propanol and diluting it to 10 ml with the same solvent. After being stirred for about 10 minutes, the solution is filtered. 1 ml of the above filtered solution is diluted to 20.0 ml with the mobile phase.

The reference solution is made by dissolving 5 mg of (S)-2-(2-oxopyrrolidin-1-yl)butanamid and 5 mg Imp D and diluting it to 5 ml with the mobile phase. This reference solution is the system suitability. A minimum resolution of 1.5 is required, between the peaks due to Imp D and (S)-2-(2-oxopyrrolidin-1-yl)butanamid.

Furthermore, to detect further typical degradation products, the detection of [(2RS)-2-(2-oxopyrrolidin-1-yl) butanoic acid called Imp A and [(2Z)-2-(2-oxopyrrolidin-1-yl) but-2-enamide called Imp B the following conditions are used. (Table III)

**Table III**

| | |
|---|---|
| Mobile phase | 1,96 g/L solution of sulfuric acid, acetonitrile (4:96 v/v) |
| Flow rate | 1 mL/min |
| Detection | Spectrophotometer at 205 nm |
| Column | Silica gel for chromatography (5 µm) l=0,25 m, ∅ =4,6 mm |
| Injection | 10 µl |
| Run Time | Twice the retention time of (S)-2-(2-oxopyrrolidin-1-yl)butanamid |
| Solvent mixure | Water, acetonitrile (4:96 v/v) |

### Preparation of standard solutions and sample

A test solution is prepared by dissolving 0.5 ml of KEPPRA solution or M mg of product which is equivalent to 50.0 mg of (S)-2-(2-oxopyrrolidin-1-yl)butanamid in the solvent mixture and diluting to 25 ml with the solvent mixure.Then, 5 mL of this solution is diluted to 50 mL with the same solvent mixture.

A reference solution (a) is prepared by dissolving 5 mg of (S)-2-(2-oxopyrrolidin-1-yl)butanamid and 5 mg of 2-pyrrolidone in the solvent mixture and diluting it to 25 mL with the solvent mixture.

A reference solution (b) is prepared by dissolving 50 mg of (S)-2-(2-oxopyrrolidin-1-yl)butanamid reference substance in the solvent mixture and diluting it to 25 ml with the solvent mixture.Then, 5 ml of the above solution are diluted to 50 ml with the same solvent mixture.

A reference solution (c) is prepared by diluting 1 ml of the test solution to 20 ml with the solvent mixture,1 ml of this solution is diluted to 100 ml with the same solvent mixture.

A reference solution (d) is prepared by dissolving 5 mg of (S)-2-(2-oxopyrrolidin-1-yl)butanamid Imp A reference substance and 5 mg (S)-2-(2-oxopyrrolidin-1-yl)butanamid Imp B reference substance in the solvent mixture and diluting it to 50 ml with the solvent mixture. 1 ml of the solution is diluted to 50 ml with the solvent mixture. Again, 5 ml of this solution are diluted to 100 m with the solvent mixture.

### Comparison of stability concerning various formulations

In accordance with the description above, various formulations have been prepared. The composition of these formulations is given in the following Table IV.

Example A is a formulation in accordance with the present invention including a pharmaceutically acceptable acid.
Example B has been provided to demonstrate that in the presence of a base agent which is necessary in an effervescent formulation (as disclosed in WO 2011/136751 A2), the stability of the formulation is affected. Similarly, the stability of a levetiracetam formulation is negatively effected if no acid is present in the formulation (Example C). Thus, the present invention is based on the surprising finding that the presence of a pharmaceutically acceptable acid in the solid formulation improves the stability of the formulation comprising levetiracetam.

**Table IV**

| **All products are packaged in alu-alu sachets.** | **Example A: Formulation of the invention.** | **Example B: Formulation with base agent. (effervescent agent)** | **Example C: Formulation without acid agent.** |
|---|---|---|---|
| **Ingredients:** | **Content:** | **Content:** | **Content:** |
| **(*S*)-2-(2-oxopyrrolidin-1-yl)butanamid** | 1000 mg | 1000 mg | 1000 mg |
| Citric acid anhydrous | 400 mg | 400 mg | ------- |
| Base agent (sodium hydrogen carbonate) | ----- | 150 mg | ----- |
| Sorbitol | 1430 mg | 1430 mg | 1430 mg |
| Polyethylene glycol 6000 | 100 mg | 100 mg | 100 mg |
| Sodium saccharinate | 20 mg | 20 mg | 20 mg |
| Lemon juice flavor | 50 mg | 50 mg | 50 mg |

In the stability measurements, the stability of Examples A through C at differenct storage conditions have been determined over up to 6 months. The results of the stability measurements are given in the following Tables V and VI.

**Table V**

| *STORAGE CONDITIONS:* 25°C± 2°C/ 60% RH ± 5% | | *PRODUCT:* | | |
|---|---|---|---|---|
| *PACKAGING:* ALU-ALU Sachets | | ***0 months*** | | |
| | | **Example A:** | **Example B:** | **Example C:** |
| **Test** | **Specification** | **Formulation of the invention.** | **Formulation with base agent.** | **Formulation without acid agent.** |
| Appearance | White granules, | White granules, | White granules, | White granules, |
| Sum of all degradation products detected (% relative areas) | ≤ 1% | 0.0 | 0.0 | 0.0 |

| | | ***3 months*** | | |
|---|---|---|---|---|
| | | **Example A:** | **Example B:** | **Example C:** |

| **Test** | **Specification** | **Formulation of the invention.** | **Formulation with base agent.** | **Formulation without acid agent.** |
|---|---|---|---|---|
| Appearance | White granules, | White granules, | White granules, | White granules, |
| Sum of all degradation products detected (% relative areas) | ≤ 1% | 0.05 | 0.1 | 0.05 |

| | | ***6 months*** | | |
|---|---|---|---|---|
| | | **Example A:** | **Example B:** | **Example C:** |
| **Test** | **Specification** | **Formulation of the invention.** | **Formulation with base agent.** | **Formulation without acid agent.** |
| Appearance | White granules, | White granules, | White granules, | White granules, |
| Sum of all degradation products detected (% relative areas) | ≤ 1% | 0.05 | 0.2 | 0.1 |

**Table VI**

| *STORAGE CONDITIONS:* 40°C± 2°C/ 75% RH ± 5% | | *PRODUCT:* | | |
|---|---|---|---|---|
| *PACKAGING:* Sachets ALU- ALU, **0 Months** | | ***0 months*** | | |
| | | **Example A:** | **Example B:** | **Example C:** |
| **Test** | **Specification** | **Formulation of the invention.** | **Formulation with base agent.** | **Formulation without acid agent.** |
| Appearance | White granules, | White granules, | White granules, | White granules, |
| Sum of all degradation products detected (% relative areas) | ≤ 1% | 0.0 | 0.0 | |

| | | ***3 months*** | | |
|---|---|---|---|---|
| | | **Example A:** | **Example B:** | **Example C:** |
| **Test** | **Specification** | **Formulation of the invention.** | **Formulation with base agent.** | **Formulation without acid agent.** |
| Appearance | White granules, | White granules, | Solid | White granules, |
| Sum of all degradation products detected (% relative areas) | ≤ 1% | 0.1 | 0.35 | 0.25 |

| | | ***6 months*** | | |
|---|---|---|---|---|
| | | **Example A:** | **Example B:** | **Example C:** |

| **Test** | **Specification** | **Formulation of the invention.** | **Formulation with base agent.** | **Formulation without acid agent.** |
|---|---|---|---|---|
| Appearance | White granules, | White granules, | Solid | White granules, |
| Sum of all degradation products detected (% relative areas) | ≤ 1% | 0.26 | 0.85 | 0.65 |

The results in the Tables above clearly demonstrate that the degradation rate of granules in accordance with the present invention is significantly lower than with the formulations according to the prior art which may include an effervescent couple (basic and acidic agents) or do not have any acid (Example C).

### List of references

US2011/0021786 A1
Handbook of Pharmaceutical Excipients, 3rd edition, page 342 & 452.

The following pages of the description refer to the embodiments of the invention listed as separate items:
1. Water-soluble granules comprising (*S*)-2-(2-oxopyrrolidin-1-yl)butanamid and at least one pharmaceutically acceptable acid.
2. The granules of item 1, wherein the acid(s) is/are a carboxylic acid(s), preferably (a) carboxylic acid(s) having from 4 to 12, more preferably 5 to 12, and even more preferably 6 to 7 carbon atoms.
3. The granules of item 1 or 2, wherein the acid(s) is/are selected from citric acid, succinic acid, tartaric acid, or any combination thereof, and is preferably citric acid.
4. The granules of any of the preceding items, wherein the acid(s) is/are present in anhydrous form.
5. The granules of any of the preceding items, comprising the acid(s) in an amount of 5 to 20 wt.-%, particularly 10 to 15 wt.-%, and more particularly 13 to 14 wt.-%.
6. The granules of any of the preceding items comprising (*S*)-2-(2-oxopyrrolidin-1-yl)butanamid in an amount of 20 to 50 wt.-%, particularly 30 to 40 wt.-%, and more particularly 33 to 35 wt.-%.
7. The granules of any of the preceding items further comprising at least one lubricant.
8. The granules of item 7, wherein the lubricant(s) is/are selected from polyethylene glycol (PEG), sodium benzoate micronized or any combination thereof.
9. The granules of item 7 or 8, wherein the lubricant(s) is/are polyethylene glycol 6000.
10. The granules of any of item 7 to 9, comprising the lubricant(s) in an amount of 1 to 10 wt.-%, particularly 2 to 5 wt.-%, and more particularly 3 to 4 wt.-%.
11. The granules of any of the preceding items, wherein the granules do not comprise maltitol.
12. The granules of any of the preceding items, further comprising at least one filler.
13. The granules of item 12, wherein the filler(s) is/are selected from sorbitol, mannitol, or any combination thereof, and is preferably sorbitol.
14. The granules of item 12 or 13, comprising the filler(s) in an amount of 35 to 60 wt.-%, particularly 40 to 55 wt.-%, and more particularly 48 to 50 wt.-%.
15. The granules of any of the preceding items, further comprising at least one sweetener.
16. The granules of item 15, wherein the sweetener(s) is/are selected from sodium saccharinate, calcium saccharinate, acesulfame potassium, sodium cyclamate, aspartame, or any combination thereof, and is preferably sodium saccharinate.
17. The granules of item 15 or 16, comprising the sweetener(s) in an amount of 0.1 to 1 wt.-%, particularly 0.6 to 0.9 wt.-%, and more particularly 0.7 to 0.8 wt.-%.
18. The granules of any of the preceding items, further comprising at least one flavoring agent.
19. The granules of item 18, wherein the flavoring agent(s) is/are selected from lemon juice flavor, cherry flavor, raspberry flavor, strawberry flavor, orange flavor, mandarin flavor, or any combination thereof, and is preferably lemon juice flavor.
20. The granules of item 18 or 19, comprising the flavoring agent(s) in an amount of 0.5 to 3 wt.-%, particularly 1 to 2 wt.-%, and more particularly 1.7 to 1.9 wt.-%.
21. The granules of any of the preceding items, wherein the granules are water soluble.
22. The granules of any of the preceding items, wherein the granules are for oral administration, preferably as oral solution.
23. The granules of any of the preceding items, wherein the granules have an average particle size distribution about 40 µm to 800 µm estimated by analytical sieving.
24. The granules of item 1, comprising or consisting of (*S*)-2-(2-oxopyrrolidin-1-yl)butanamid, a pharmaceutically acceptable acid, a filler, a lubricant, a sweetener, and a flavoring agent.
25. The granules of item 1, comprising or consisting of (*S*)-2-(2-oxopyrrolidin-1-yl)butanamid, anhydrous citric acid, sorbitol, polyethylene glycol 6000, sodium saccharinate, and lemon juice flavor.
26. The granules of item 1, consisting of 33.3 wt.-% (*S*)-2-(2-oxopyrrolidin-1-yl)butanamid, 13.3 wt.-% anhydrous citric acid, 47.7 wt.-% sorbitol, 3.3 wt.-% polyethylene glycol 6000, 0.7 wt.-% sodium saccharinate, and 1.7 wt.-% lemon juice flavor.
27. Granules of any of items 1 to 26 for use in a medical treatment, in particular for use in a method for treating epilepsy.
28. Granules of any of items 1 to 26 for use as an antiepileptic drug.
29. A method for producing the granules of any of items 1 to 26, comprising combining the components of the granules.
30. The method of item 29, further comprising filling the combined components in at least one container under an inert gas atmosphere.
31. The method of item 30, wherein the container is selected from a sachet, a case, a tube, a sleeve, a bag, a bottle, an ampoule, a blister, a cap, or a foil, and is preferably a sachet.
32. The method of item 30 or 31, wherein the inert gas is select from nitrogen gas, helium gas, argon gas, or any combination thereof.
33. The method of any of items 29 to 32, wherein the components are combined in one combining step.
34. The method of any of items 29 to 32, wherein the components are combined in at least two consecutive combining steps, particularly in three consecutive combining steps.
35. The method of any of items 30 to 32, comprising
   a first combining step, in which (*S*)-2-(2-oxopyrrolidin-1-yl)butanamid, the at least one pharmaceutically acceptable acid, preferably as defined in any of items 2 to 5, and optionally a filler, preferably as defined in item 13 or 14, are combined to provide a first mixture;
   an optional second combining step, in which the first mixture is combined with at least one flavoring agent, preferably as defined in item 19 or 20, and/or with at least one sweetener, preferably as defined in item 16 or 17, to provide a second mixture; and
   an optional third combining step, in which the second mixture is combined with at least one lubricant, preferably as defined in any of items 8 to 10.
36. The method of item 35, wherein the combining in the first combining step takes 10 to 30 min, particularly 15 to 25 min, and more particularly 20 to 22 min.
37. The method of item 35 or 36, wherein the (*S*)-2-(2-oxopyrrolidin-1-yl)butanamid, the at least one pharmaceutically acceptable acid and the filler are sieved before they are combined in the first combining step, particularly through a 20 mesh sieve.
38. The method of any of items 35 to 38, wherein the combining in the second combining step takes 5 to 25 min, particularly 10 to 20 min, and more particularly 15 to 17 min.
39. The method of item any of items 35 to 38, wherein the flavoring agent and the sweetener are sieved before they are combined in the second combining step, particularly through a 40 mesh sieve.
40. The method of any of items 35 to 39, wherein the combining in the third combining step takes 1 to 10 min, particularly 3 to 8 min, and more particularly 5 to 7 min.
41. The method of any of items 35 to 40, wherein the combining is carried out by mixing.
42. The method of item 41, wherein the mixing is carried out at 40 rpm.

## Claims

1. Solid pharmaceutical composition comprising non-effervescent and water soluble granules which comprise (*S*)-2-(2-oxopyrrolidin-1-yl)butanamid and a pharmaceutically acceptable acid.

2. The solid pharmaceutical composition of claim 1 wherein the pharmaceutically acceptable acid is a water soluble acid.

3. The solid pharmaceutical composition of claim 1 or 2 wherein the acid(s) is/are a carboxylic acid(s), preferably (a) carboxylic acids(s) having from 4 to 12, more preferably 5 to 12, and even more preferably 6 to 7 carbon atoms.

4. The solid pharmaceutical composition of claim 3 wherein the acid(s) is/are selected from citric acid, succinic acid, tartaric acid, or any combination thereof, and is preferably citric acid.

5. The solid pharmaceutical composition of any of the preceding claims, wherein the acid(s) is/are present in anhydrous form.

6. The solid pharmaceutical composition of any of the preceding claims, wherein the granules do not comprise maltitol.

7. The solid pharmaceutical composition of any of the preceding claims, further comprising at least one sweetener.

8. The solid pharmaceutical composition of claim 7, wherein the sweetener(s) is/are selected from sodium saccharinate, calcium saccharinate, acesulfame potassium, sodium cyclamate, aspartame, or any combination thereof, and is preferably sodium saccharinate.

9. The solid pharmaceutical composition of any of the preceding claims, wherein the granules are water soluble.

10. The solid pharmaceutical composition of any of the preceding claims, wherein the granules are for oral administration, preferably as oral solution.

11. The solid pharmaceutical composition of any of the preceding claims, wherein the granules have an average particle size distribution of about 40 µm to 800µm estimated by analytical sieving.

12. The solid pharmaceutical composition of claim 1 wherein the granules consist of (*S*)-2-(2-oxopyrrolidin-1-yl)butanamid, a pharmaceutically acceptable acid, a filler, a lubricant, a sweetener, and a flavoring agent.

13. The solid pharmaceutical composition of any of claims 1 to 10 for use in a medical treatment, in particular for use in a method for treating epilepsy.

14. A method for producing a solid pharmaceutical composition of any of claims 1 to 13, comprising combining the components of the granules and optionally granulating said components.

15. A method of claim 14, wherein the (*S*)-2-(2-oxopyrrolidin-1-yl)butanamid, the at least one pharmaceutically acceptable acid and a filler are sieved before they are combined in a first combining step, particularly through a 20 mesh sieve.

## Patentansprüche

1. Feste pharmazeutische Zusammensetzung, umfassend nichtsprudelnde und wasserlösliche Körnchen, die *(S)-*2-(2-Oxopyrrolidin-1-yl)butanamid und eine pharmazeutisch verträgliche Säure umfassen.

2. Feste pharmazeutische Zusammensetzung nach Anspruch 1, wobei die pharmazeutisch verträgliche Säure eine wasserlösliche Säure ist.

3. Feste pharmazeutische Zusammensetzung nach Anspruch 1 oder 2, wobei die Säure(n) (eine) Carbonsäure(n), vorzugsweise (eine) Carbonsäure(n) mit von 4 bis 12, stärker bevorzugt 5 bis 12, und noch stärker bevorzugt 6 bis 7 Kohlenstoffatome ist/sind.

4. Feste pharmazeutische Zusammensetzung nach Anspruch 3, wobei die Säure(n) ausgewählt ist/sind aus Zitronensäure, Bernsteinsäure, Weinsäure, oder einer beliebigen Kombination davon, und ist bevorzugt Zitronensäure.

5. Feste pharmazeutische Zusammensetzung nach einem beliebigen der vorhergehenden Ansprüche, wobei die Säure(n) in wasserfreier Form vorhanden ist/sind.

6. Feste pharmazeutische Zusammensetzung nach einem beliebigen der vorhergehenden Ansprüche, wobei die Körnchen nicht Maltitol umfassen.

7. Feste pharmazeutische Zusammensetzung nach einem beliebigen der vorhergehenden Ansprüche, des Weiteren umfassend mindestens ein Süßungsmittel.

8. Feste pharmazeutische Zusammensetzung nach Anspruch 7, wobei das/die Süßungsmittel ausgewählt ist/sind aus Natriumsaccharinat, Calciumsaccharinat, Kalium Acesulfam, Natriumcyclamat, Aspartam, oder einer beliebigen Kombination davon, und vorzugsweise Natriumsaccharinat ist.

9. Feste pharmazeutische Zusammensetzung nach einem beliebigen der vorhergehenden Ansprüche, wobei die Körnchen wasserlöslich sind.

10. Feste pharmazeutische Zusammensetzung nach einem beliebigen der vorhergehenden Ansprüche, wobei die Körnchen zur oralen Verabreichung sind, vorzugsweise als orale Lösung.

11. Feste pharmazeutische Zusammensetzung nach einem beliebigen der vorhergehenden Ansprüche, wobei die Körnchen eine durchschnittliche Partikelgrößenverteilung von etwa 40 µm bis 800 µm, abgeschätzt durch analytisches Sieben, aufweisen.

12. Feste pharmazeutische Zusammensetzung nach Anspruch 1, wobei die Körnchen aus *(S)-*2-(2-Oxopyrrolidin-1-yl)butanamid, einer pharmazeutisch verträglichen Säure, einem Füllstoff, einem Schmiermittel, einem Süßungsmittel und einem Geschmacksstoff besteht.

13. Feste pharmazeutische Zusammensetzung gemäß einem beliebigen der Ansprüche 1 bis 10, zur Verwendung in einer medizinischen Behandlung, insbesondere zur Verwendung in einem Verfahren zum Behandeln von Epilepsie.

14. Verfahren zum Herstellen einer festen pharmazeutischen Zusammensetzung gemäß einem beliebigen der Ansprüche 1 bis 13, umfassend das Kombinieren der Bestandteile der Körnchen und gegebenenfalls Granulieren der Bestandteile.

15. Verfahren nach Anspruch 14, wobei das *(S)-*2-(2-Oxopyrrolidin-1-yl)butanamid, die mindestens eine pharmazeutisch verträgliche Säure und ein Füllstoff gesiebt werden, bevor sie in einem ersten Kombinierungsschritt kombiniert werden, insbesondere durch ein Siebweite 20 Sieb.

## Revendications

1. Composition pharmaceutique solide comprenant des granules non effervescents et solubles dans l'eau qui comprennent du (S)-2-(2-oxopyrrolidin-1-yl)butanamide et un acide pharmaceutiquement acceptable.

2. Composition pharmaceutique solide selon la revendication 1, dans laquelle l'acide pharmaceutiquement acceptable est un acide soluble dans l'eau.

3. Composition pharmaceutique solide selon la revendication 1 ou 2, dans laquelle l'acide (les acides) est/sont un (des) acide(s) carboxylique(s), de préférence un (des) acide(s) carboxylique(s) ayant 4 à 12, plus préférablement 5 à 12, et de manière davantage encore préférée 6 à 7 atomes de carbone.

4. Composition pharmaceutique solide selon la revendication 3, dans laquelle l'acide (les acides) est/sont choisi(s) parmi l'acide citrique, l'acide succinique, l'acide tartrique, ou n'importe quelle combinaison de ceux-ci, et est de préférence l'acide citrique.

5. Composition pharmaceutique solide selon l'une quelconque des revendications précédentes, dans laquelle l'acide (les acides) est/sont présents sous forme anhydre.

6. Composition pharmaceutique solide selon l'une quelconque des revendications précédentes, dans laquelle les granulés ne comprennent pas de maltitol.

7. Composition pharmaceutique solide selon l'une quelconque des revendications précédentes, comprenant en outre au moins un édulcorant.

8. Composition pharmaceutique solide selon la revendication 7, dans laquelle l'édulcorant (les édulcorants) est/sont choisi(s) parmi le saccharinate de sodium, le saccharinate de calcium, l'acésulfame de potassium, le cyclamate de sodium, l'aspartame, ou n'importe quelle combinaison de ceux-ci et est de préférence le saccharinate de sodium.

9. Composition pharmaceutique solide selon l'une quelconque des revendications précédentes, dans laquelle les granules sont solubles dans l'eau.

10. Composition pharmaceutique solide selon l'une quelconque des revendications précédentes, dans laquelle les granules sont destinés à une administration orale, de préférence sous forme de solution buvable.

11. Composition pharmaceutique solide selon l'une quelconque des revendications précédentes, dans laquelle les granules ont une distribution de la taille moyenne des particules d'environ 40 µm à 800 µm, estimée par tamisage analytique.

12. Composition pharmaceutique solide selon la revendication 1, dans laquelle les granules sont constitués de (S)-2-(2-oxopyrrolidin-1-yl)butanamide, d'un acide pharmaceutiquement acceptable, d'une charge, d'un lubrifiant, d'un édulcorant et d'un agent aromatisant.

13. Composition pharmaceutique solide selon l'une quelconque des revendications 1 à 10 destinée à une utilisation dans un traitement médical, en particulier destinée à une utilisation dans une méthode pour traiter l'épilepsie.

14. Procédé de production d'une composition pharmaceutique solide selon l'une quelconque des revendications 1 à 13, comprenant la combinaison des constituants des granules et éventuellement la granulation desdits constituants.

15. Procédé selon la revendication 14, dans lequel le (S)-2-(2-oxopyrrolidin-1-yl)butanamide, le ou les acide(s) pharmaceutiquement acceptable(s) et une charge sont tamisés avant qu'ils soient combinés dans une première étape de combinaison, en particulier à travers un tamis de 20 mesh.
